# EUROPEAN PATENT SPECIFICATION

(11) **EP 1 339 416 B1**
(45) Date of publication and mention of the grant of the patent: **07.11.2012**
(21) Application number: 01958270.9
(22) Date of filing: 17.08.2001
(51) Int. Cl.: A61K 33/04, A61K 33/00, A61K 47/00, A23K 1/175

(54) **TRACE ELEMENTS**
SPURENELEMENTE
OLIGO-ELEMENTS

(30) Priority: 28.08.2000 ZA 200004456
(43) Date of publication of application: 03.09.2003
(73) Proprietor: Warburton Technology Limited, Dublin 4 (IE)
(72) Inventor: LAURIE, Robert, Naylor, 7130 Somerset West (ZA); VOSLOO, Lambertus, Petrus, 7612 Stellenbosch (ZA)
(74) Representative: Tetzner, Michael
(86) International application number: PCT/IB2001/001486
(87) International publication number: WO 2002/017933

(56) References cited:
- WO-A-83/01559
- DE-A- 4 128 760
- DE-A- 19 617 185
- US-A- 4 335 116
- DATABASE WPI Week 198344, Derwent Publications Ltd., London, GB; Class B05, AN 1983-808091, XP002958133 & ZA 8 206 778 A (ANIMALIA PTY LTD) 13 June 1983
- TRENGOVE C.L. & JUDSON G.J.: 'Trace element supplementation of sheep: evaluation of various copper supplements and a soluble glass bullet containing copper, cobalt and selenium' AUSTRALIAN VETERINARY JOURNAL vol. 62, no. 10, 1985, pages 321 - 324, XP002968743
- KOENIG K.M. ET AL: 'Effects of diet and chemical form of selenium on selenium metabolism in sheep' J. ANIM. SCI. vol. 75, 1997, pages 817 - 827, XP002958134
- ALLEN W.M. & MALLINSON C.B.: 'Parental methods of supplementation with copper and selenium' THE VETERINARY RECORD vol. 114, 1984, pages 451 - 454, XP002968744
- EDMISTON F.L. & BULL R.C.: 'Evaluation of selenium reticular pellets and injectable copper edetate for yearling replacement heifers' PROCEEDINGS, ANNUAL MEETING - WESTERN SECTION, AMERICAN SOCIETY OF ANIMAL SCIENCE vol. 39, 1988, pages 111 - 114, XP002968745

## Description

It has been found that there is a deficiency of certain trace elements in pastures for livestock in particular areas in South Africa and also in other countries. Various suggestions have been made to provide the required trace elements to such animals. Different chemical compounds and complexes have been investigated for applying the trace elements by way of licks, drenches or injections.

In general the problem with injectable solutions is that there are too low concentrations of the minerals in the solutions. This means that relatively large quantities have to be injected, which in turn cause tissue damage and also abscesses at the site of injection. Furthermore, it is generally the case that different trace elements seldomly are individually sufficient. This means that two or more trace element solutions have to be provided by way of separate injections.

US 4,335,116 discloses mineral-containing therapeutic compositions containing EDTA complexes of trace elements. EDTA means ethylene diaminotetraacetic acid (C₁₀H₁₆O₈N₂ or (HO₂CH₂C)₂NCH₂CH₂N(CH₂CO₂H)₂). Notably, US 4,335,116 utilises tetra-sodium EDTA, a selenium glycine complex, and metal chlorides for the preparation of the EDTA complexes. Unfortunately, the chloride ions cause contamination and each complex solution is to be made individually. Furthermore, overnight time is required for complexing and heating up afterward to speed up the process requires extra apparatus. If mixtures are required, the individual solutions are to be blended. If various concentrations as well as compositions are to be made, it can only be done in a cumbersome way, requiring extra apparatus. A further problem may arise when mixtures of high concentration are needed. In certain cases it would be impossible to deliver them, because mixing is always accompanied by dilution. Due to the separate preparation of the EDTA-complexes the total metal concentration in any mixture of said complex solutions is rather low.

WO 83/01559 discloses a process in which amoung other components EDTA sodium salt and selenium is used for the preparation of a composition suitable for promoting the utilization of fodder by the animals. All components are separately prepared and finally combined.

Koenig et al. in J. Anim. Sci. 1997, vol 75, pages 817-827 describe effects of diet and chemical form of selenium on selenium metabolism in sheep. Cobalt-EDTA was prepared as the lithium salt and elemental selenium was dissolved in nitric acid.

It is an object of the invention to provide a method which permits to prepare a substantially contamination free injectable trace element solution with high metal concentration, said method requiring a minimum of process effort and apparatus. Furthermore, it is also an object of the invention to provide an injectable trace element solution with high metal concentration which is substantially free of contamination and which can be manufactured with a minimum of process effort and apparatus.

According to the invention a method of preparing an injectable trace element solution is characterized in that it includes the steps of
(a) preparing more than one EDTA-complex in the same container in a single continuous process by using disodium EDTA and adding metal oxides, metal hydroxides or metal carbonates; and
(b) adding sodium selenite to the EDTA-complexes.

Furthermore, according to the invention an injectable trace element solution is characterized in that it includes
(a) more than one EDTA-complex prepared by using disodium EDTA and adding metal oxides, metal hydroxides or metal carbonates; and
(b) sodium selenite.

The EDTA-complexes include metal compounds selected from the group consisting of copper, manganese, zinc, molybdenum and chromium.

The invention will now be described by way of an example of preparing an injectable solution in accordance with the invention.

### EXAMPLE:

1. 90,37g EDTA is suspended in a quantity of distilled water at 50°C and is stirred continuously. In small proportions firstly 24,74g sodium hydroxide (NaOH) and then 25,16g zinc oxide (ZnO) are added in sequence (such that disodium EDTA is obtained from EDTA and NaOH and ZnO is added to this disodium EDTA).
2. To this are added (in the same container) according to the same method as described in 1) the following chemicals: 106,39g EDTA, 29,12g NaOH, and 45,45g manganese carbonate (MnCO₃.xH₂O).
3. To this are added according to the same method as described in 1) the following chemicals: 45,99g EDTA, 12,59g NaOH, and 18,81g basic copper carbonate (CuCO₃Cu(OH)₂. H₂O).
4. At this stage the pH is brought to 7, if necessary, by either the addition of NaOH (if acid) or EDTA (if alkaline).
5. Subsequently to this are added according to the same method as described in 1) the following chemicals: 31,59g EDTA, 15,38g NaOH, and 25,62g chromium tri-chloride hexahydrate (CrCl₃.6H₂O).
6. Lastly 12,09g sodium selenite is added.
7. Finally the total volume is adapted by the addition of distilled water.
8. Filtration takes place.

If 25,16g zinc oxide, 45,45g manganese carbonate, 18,81g basic copper carbonate, 25,62g chromium tri-chloride hexahydrate, 12,09g sodium selenite, 274,34g EDTA and 81,83g NaOH are used, and if the total volume is 1 litre, then the zinc concentration will be 20mg/ml, the manganese concentration 20mg/ml, copper concentration 10mg/ml, the chromium concentration 5mg/ml and the selenium concentration 5mg/ml (the total concentration of the metals and the selenium in the trace element solution, therefore, is 60 mg/ml).

In the above example the order of mixing the chemicals may be changed to some extent without any influence on the products formed.

The above product can be obtained as solid by evaporation of the appropriate solution.

All of the above-mentioned chemicals may be substituted by others, provided the substitute are used in equivalent quantities. The particulars are as follows:
1. Basic zinc carbonate (2ZnCO₃·3Zn(OH)₂) or zinc hydroxide (Zn(OH)₂) in place of zinc oxide.
2. Manganese hydroxide (Mn(OH)₂) in place of manganese carbonate.
3. Cupric hydroxide (Cu(OH)₂) or cupric oxide (CuO) in place of basic copper carbonate.
4. Anhydrous chromium tri-chloride (CrCl₃) in place of chromium tri-chloride hexahydrate.

Regarding molybdenium sodium molybdate (Na₂MoO₄) or molybdenum tri-oxide can be used.

## Claims

1. A method of preparing an injectable trace element solution, **characterized in that** it includes the steps of
(a) preparing more than one EDTA-complex in the same container in a single continuous process by using disodium EDTA and adding metal oxides, metal hydroxides or metal carbonates; and
(b) adding a sodium selenite solution to the EDTA-complexes.

2. A method as claimed in claim 1, **characterized in that** the EDTA-complexes include metal compounds selected from the group consisting of copper, manganese, zinc, molybdenum and chromium.

3. A method as claimed in claim 1 and 2, **characterized in that** the EDTA-complexes include metal compounds of copper, manganese, zinc, and chromium in amounts such that after final addition of water the total concentration of the metals and the selenium in the trace element solution is 60 mg/ml, and **in that** the trace element solution includes:
(a) 20 mg/ml zinc,
(b) 20 mg/ml manganese,
(c) 10 mg/ml copper,
(d) 5 mg/ml chromium, and
(e) 5 mg/ml selenium.

4. An injectable trace element solution, **characterized in that** it includes
(a) more than one EDTA-complex prepared by using disodium EDTA and adding metal oxides, metal hydroxides or metal carbonates; and
(b) sodium selenite,
and **in that** the EDTA-complexes include metal compounds of copper, manganese, zinc, and chromium in amounts such that the total concentration of the metals and the selenium in the trace element solution is 60 mg/ml, and **in that** the trace element solution includes:
(a) 20 mg/ml zinc,
(b) 20 mg/ml manganese,
(c) 10 mg/ml copper,
(d) 5 mg/ml chromium, and
(e) 5 mg/ml selenium.

## Patentansprüche

1. Verfahren zur Herstellung einer injizierbaren Spurenelementlösung, **dadurch gekennzeichnet, dass** es die folgenden Schritte enthält:
(a) Herstellen von mehr als einem EDTA-Komplex im selben Behälter in einem einzelnen kontinuierlichen Prozess unter Verwendung von Dinatrium-EDTA und Hinzufügen von Metalloxiden, Metallhydroxiden oder Metallcarbonaten und
(b) Hinzufügen einer Natriumselenitlösung zu den EDTA-Komplexen.

2. Verfahren nach Anspruch 1, **dadurch gekennzeichnet, dass** die EDTA-Komplexe Metallverbindungen enthält, ausgewählt aus der Gruppe, die aus Kupfer, Mangan, Zink, Molybdän und Chrom.

3. Verfahren nach Anspruch 1 und 2, **dadurch gekennzeichnet, dass** die EDTA-Komplexe Metallverbindungen von Kupfer, Mangan, Zink und Chrom in derartigen Mengen enthält, dass nach finaler Zugabe von Wasser die Gesamtkonzentration der Metalle und des Selens in der Spurenelementlösung 60 mg/ml beträgt, und dadurch, dass die Spurenelementlösung enthält:
(a) 20 mg/ml Zink,
(b) 20 mg/ml Mangan,
(c) 10 mg/ml Kupfer,
(d) 5 mg/ml Chrom und
(e) 5 mg/ml Selen.

4. Injzierbare Spurenelementlösung, **dadurch gekennzeichnet, dass** sie enthält:
(a) mehr als einen EDTA-Komplex, hergestellt unter Verwendung von Dinatrium-EDTA und Hinzufügen von Metalloxiden, Metallhydroxiden oder Metallcarbonaten und
(b) Natriumselenit,
und dadurch, dass die EDTA-Komplexe Metallverbindungen von Kupfer, Mangan, Zink und Chrom in derartigen Mengen enthält, dass die Gesamtkonzentration der Metalle und des Selens in der Spurenelementlösung 60 mg/ml beträgt, und dadurch, dass die Spurenelementlösung enthält:
(a) 20 mg/ml Zink,
(b) 20 mg/ml Mangan,
(c) 10 mg/ml Kupfer,
(d) 5 mg/ml Chrom und
(e) 5 mg/ml Selen.

## Revendications

1. Procédé de préparation d'une solution injectable d'oligoéléments, **caractérisé en ce qu'**il comprend les étapes de
(a) préparation de plus d'un complexe de l'EDTA dans le même récipient selon un procédé continu unique en utilisant l'EDTA disodique et des oxydes métalliques, des hydroxydes métalliques ou des carbonates métalliques, et
(b) addition d'une solution de sélénite de sodium aux complexes de l'EDTA.

2. Procédé selon la revendication 1, **caractérisé en ce que** les complexes de l'EDTA comprennent des composés métalliques sélectionnés parmi le groupe consistant en le cuivre, le manganèse, le zinc, le molybdène et le chrome.

3. Procédé selon les revendications 1 et 2, **caractérisé en ce que** les complexes de l'EDTA comprennent des composés métalliques du cuivre, du manganèse, du zinc et du chrome en des quantités telles qu'après l'addition finale de l'eau, la concentration totale des métaux et du sélénium dans la solution d'oligoéléments se situe à 60 mg/ml, et **en ce que** la solution d'oligoéléments contient :
(a) 20 mg/ml de zinc,
(b) 20 mg/ml de manganèse,
(c) 10 mg/ml de cuivre,
(d) 5 mg/ml de chrome, et
(e) 5 mg/ml de sélénium.

4. Solution injectable d'oligoéléments, **caractérisée en ce qu'**elle comprend
(a) plus d'un complexe de l'EDTA préparés en utilisant l'EDTA disodique et des oxydes métalliques, des hydroxydes métalliques ou des carbonates métalliques, et
(b) le sélénite de sodium,
et **en ce que** les complexes de l'EDTA comprennent des composés métalliques du cuivre, du manganèse, du zinc et du chrome en des quantités telles que la concentration totale des métaux et du sélénium dans la solution d'oligoéléments se situe à 60 mg/ml, et **en ce que** la solution d'oligoéléments contient :
(a) 20 mg/ml de zinc,
(b) 20 mg/ml de manganèse,
(c) 10 mg/ml de cuivre,
(d) 5 mg/ml de chrome, et
(e) 5 mg/ml de sélénium.
